# EUROPEAN PATENT SPECIFICATION

(11) **EP 3 097 877 B1**
(45) Date of publication and mention of the grant of the patent: **17.04.2019**
(21) Application number: 15170000.2
(22) Date of filing: 29.05.2015
(51) Int. Cl.: A61B 17/70

(54) **SPRING ELASTIC DEVICE THAT LINKS FIXED COMPONENTS ON MORE THAN TWO LEVELS OF BONE**
FEDERELASTISCHE VORRICHTUNG ZUR VERBINDUNG FESTER KOMPONENTEN AUF MEHR ALS ZWEI KNOCHENEBENEN
DISPOSITIF ÉLASTIQUE À RESSORT RELIANT DES COMPOSANTS FIXES SUR PLUS DE DEUX NIVEAUX D'OS

(43) Date of publication of application: 30.11.2016
(73) Proprietor: Mega Spine Medical Co., Ltd., New Taipei City 241 (TW)
(72) Inventor: Tung, Jung-Tsou, Taipei City (TW); Lee, Chia-Ching, 737 Tainan City (TW); Lee, Te-Jung, 802 Kaohsiung City (TW); Lo, Chia-Jui, 320 Taoyuan City (TW); Tung, Hao-Yun, 103 Taipei City (TW)
(74) Representative: Lang, Christian

(56) References cited:
- US-A1- 2006 129 147
- US-A1- 2008 033 435
- US-A1- 2008 097 431
- US-A1- 2009 048 631
- US-A1- 2009 163 953

## Description

### BACKGROUND OF THE INVENTION

### 1. Field of the Invention

The invention relates to bone fastening devices and more particularly to a spring elastic device that links fixed components on more than two levels of bone of vertebral column.

### 2. Description of Related Art

Bone fastening devices are known in the art. For example the document US 2006/0129147 A1 discloses a conventional stabilization device for bones or vertebrae and from document US 2008/0097431 A1 another conventional device for spinal stabilization is known. A conventional bone fastening device includes a flexible rod made of resilient material, and a bone fastening member for securing the rod to the vertebral column. The flexible rod is made of bio-compatible polymeric material such as polyurethane (PU). The flexible rod has a wavy surface extending lengthwise.

The flexible rod is formed of injection molding involving steps of melting plastic material and injecting the molten into a mold. The polymers of the produced rod are bonded with particles filled in. Also, links are interconnected. The produced rod includes isotropic polymeric links and thus is not homogeneous. There are defects in the polymeric structure. Pressure is applied onto the surface of the rod when it is secured to a bone fastening member. A local flow of the material of the rod may occur in the pressure application. However, the local flow of the material can loosen the bone fastening member.

There is another conventional rod fastening device including a hard section, an adjacent flexible section, and a channel through the hard and flexible sections. A core tube is inserted into the channel. At least one end of the core tube is free to move in the channel when the flexible section extends or retracts. However, the device has the following disadvantages: The number of the components is large so that the probability of malfunction is relatively high. The flexible section is hollow and some portions thereof are thin. And in turn, the thick portions thereof are possible of breaking. Also, the core tube may break or permanently deform. Thus, the flexible section may not return to its original shape. It is only applicable to a patient who had injured two-bone. Further, at most 1.5mm extension and at most 0.5mm contraction are possible. Hence, its applications are limited. For example, the document US 2009/0163953 A1 discloses such a rod assembly for spinal stabilization, which comprises a longitudinal core extending through the flexible section, wherein the end portions of the core are movably received within adapters at the end faces of the flexible section. Thus, the need for improvement still exists.

### SUMMARY OF THE INVENTION

It is therefore one object of the invention to provide a spring elastic device comprising at least one resilient connecting member; and at least one threaded connecting rod; wherein each of the at least one resilient connecting member includes an externally threaded cylinder having an axial hole with an internally threaded portion formed at an open end of the axial hole; and wherein each of the at least one threaded connecting rod includes a first externally threaded portion at a first end.

The above and other objects, features and advantages of the invention will become apparent from the following detailed description taken with the accompanying drawings.

### BRIEF DESCRIPTION OF THE DRAWINGS

FIG. 1 is a perspective view of a spring elastic device;
FIG. 2 is an exploded, lengthwise section view of the device shown in FIG. 1;
FIG. 2A is a detailed view of the area in circle A of FIG. 2;
FIG. 3 a perspective view of a spring elastic device according to a first preferred embodiment of the invention;
FIG. 4 is a lengthwise section view of the device shown in FIG. 3;
FIG. 4A is a detailed view of the area in circle B of FIG. 4;
FIG. 4B is a detailed view of the area in circle C of FIG. 4;
FIG. 5 a perspective view of a spring elastic device according to a second preferred embodiment of the invention;
FIG. 6 is a lengthwise section view of the device shown in FIG. 5;
FIG. 6A is a detailed view of the area in circle D of FIG. 6;
FIG. 6B is a detailed view of the area in circle E of FIG. 6;
FIG. 7 is an exploded view of the device shown in FIG. 5;
FIG. 8 is a side elevation of two fastening members mounted on one resilient connecting member and one threaded connecting rod of a spring elastic device;
FIG. 9 is a side elevation of three fastening members mounted on the device of the first preferred embodiment; and
FIG. 10 is a perspective view of four fastening members mounted on the device of the second preferred embodiment.

### DETAILED DESCRIPTION OF THE INVENTION

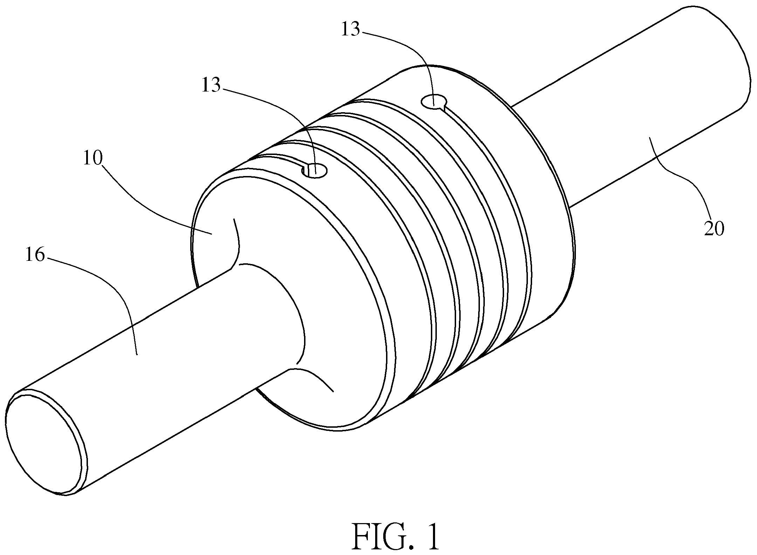

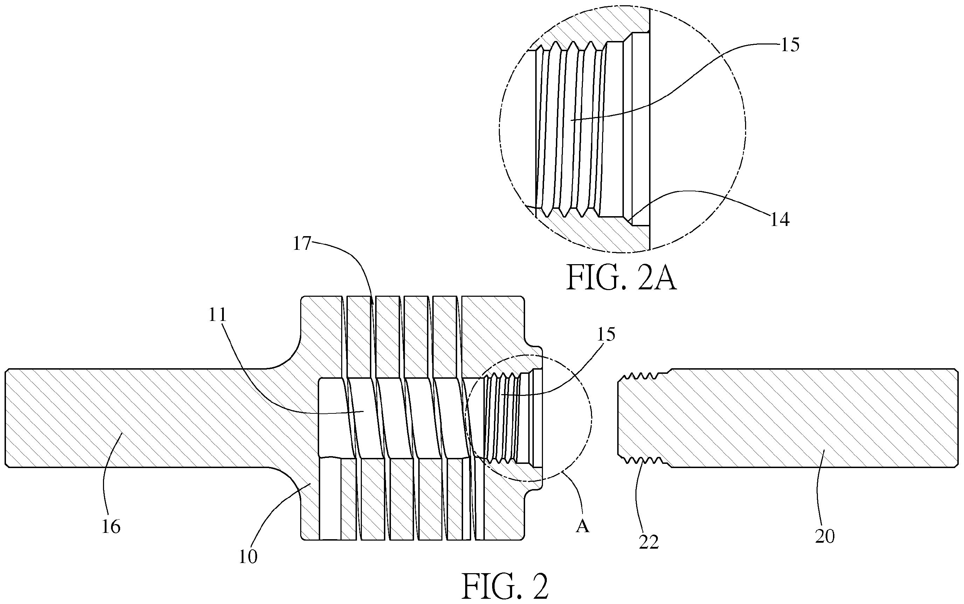

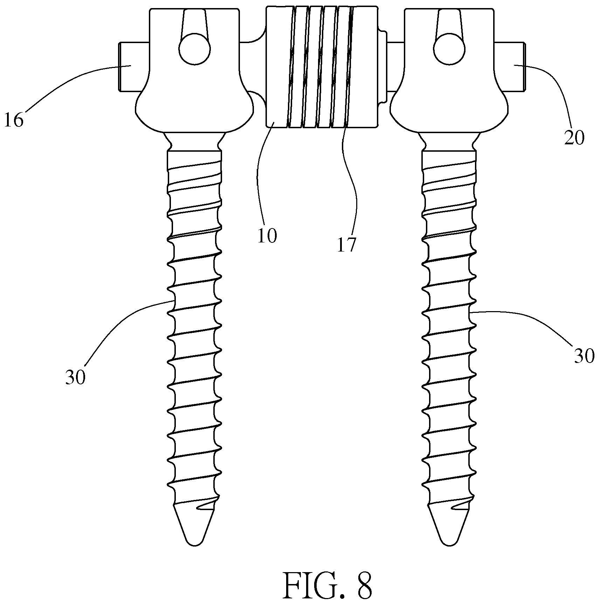

Referring to FIGS. 1, 2 and 8, one resilient connecting member 10 and one threaded connecting rod 20 of a spring elastic device are illustrated. The resilient connecting member 10 includes an externally threaded cylinder 17 having two radial holes 13 at two ends of the threads respectively, an axial hole 11 having a flat or inclined bottom, an internally threaded portion 15 formed at an open end of the axial hole 11, and a cylindrical connection rod 16 integrally formed with the externally threaded cylinder 17 and projecting out of an end of the externally threaded cylinder 17 opposing the open end of the axial hole 11.

The threaded connecting rod 20 includes an externally threaded portion 22 at one end. The externally threaded portion 22 is secured to the internally threaded portion 15 to fasten the threaded connecting rod 20 and the resilient connecting member 10 together. Two fastening members 30 are put on the threaded connecting rod 20 and the connection rod 16 respectively.

Each of the threaded connecting rods 20 and the connection rod 16 has a diameter of 5.5mm. The threaded connecting rod 20 is solid as shown or hollow in another embodiment. The externally threaded cylinder 17 has a uniform diameter and has a diameter greater than that of the threaded connecting rod 20 or that of the connection rod 16. The mouth 14 of the internally threaded portion 15 has a diameter gradually increased toward an open end of the externally threaded portion 15.

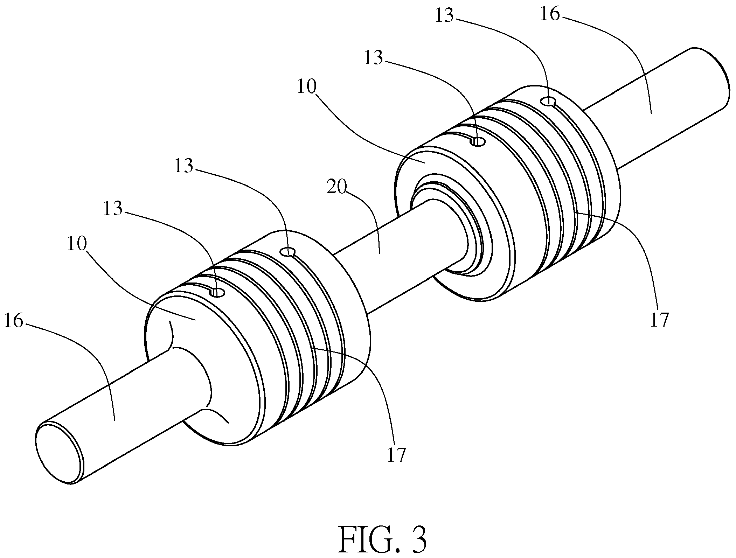

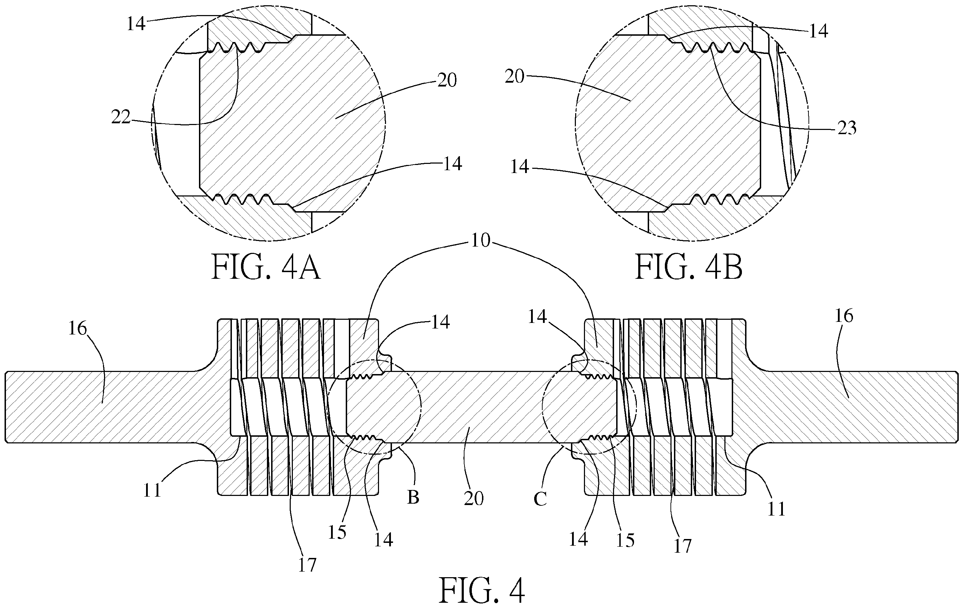

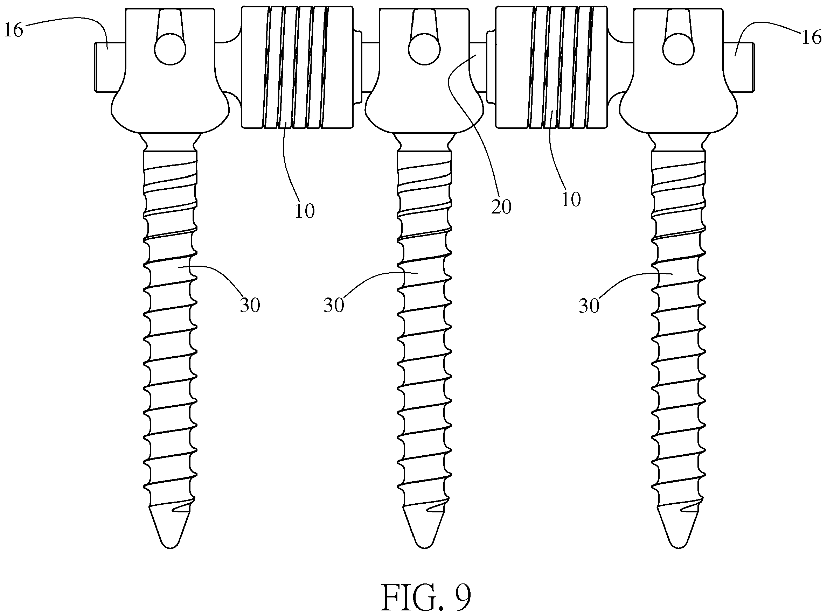

Referring to FIGS. 3, 4 and 9, a spring elastic device in accordance with a first preferred embodiment of the invention is shown. The characteristics of the resilient connecting members 10 and the threaded connecting rod 20in the first preferred embodiment are substantially the same as that previously described. In detail, the first preferred embodiment comprises two resilient connecting members 10 and a threaded connecting rod 20. Each resilient connecting member 10 includes an externally threaded cylinder 17 having two radial holes 13 at two ends of the threads respectively, an axial hole 11 having a flat or inclined bottom, an internally threaded portion 15 formed at an open end of the axial hole 11, and a cylindrical connection rod 16 integrally formed with the externally threaded cylinder 17 and projecting out of an end of the externally threaded cylinder 17 opposing the open end of the axial hole 11.

The threaded connecting rod 20 includes a first externally threaded portion 22 at a first end, and a second externally threaded portion 23 at a second end opposing the first end. The first externally threaded portion 22 is secured to the internally threaded portion 15 of one resilient connecting member 10, and the second externally threaded portion 23 is secured to the internally threaded portion 15 of the other resilient connecting member 10 respectively to fasten the threaded connecting rod 20 and the resilient connecting members 10 together. Three fastening members 30 are put on the threaded connecting rod 20, the connection rod 16 of one resilient connecting member 10, and the connection rod 16 of the other resilient connecting member 10 respectively.

Each of the threaded connecting rod 20 and the connection rod 16 have a diameter of 5.5mm. The threaded connecting rod 20 is solid as shown or hollow in another embodiment. The externally threaded cylinder 17 has a uniform diameter and has a diameter greater than that of the threaded connecting rod 20 or that of the connection rod 16. The mouth 14 of the internally threaded portion 15 has a diameter gradually increased toward an open end of the externally threaded portion 15.

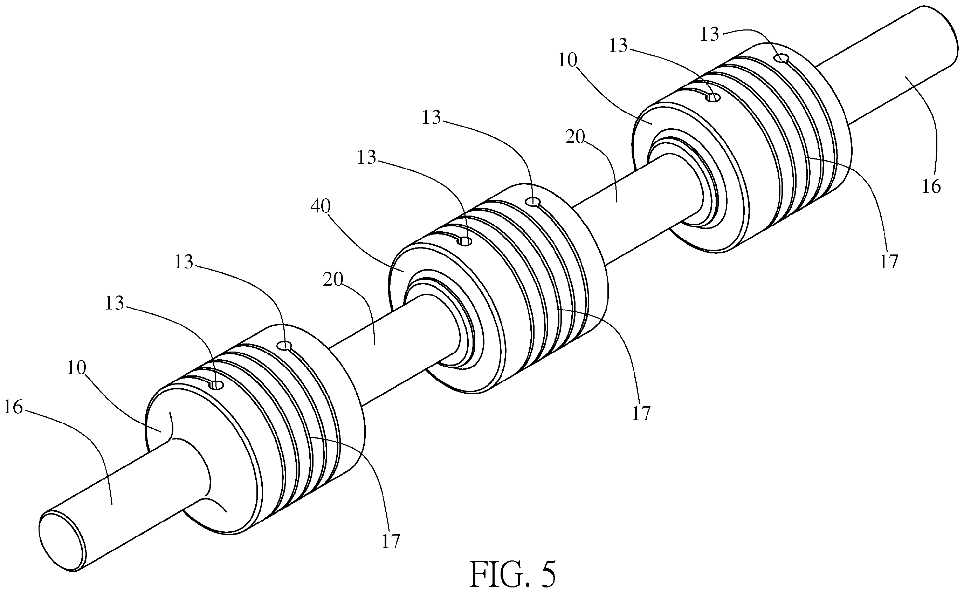

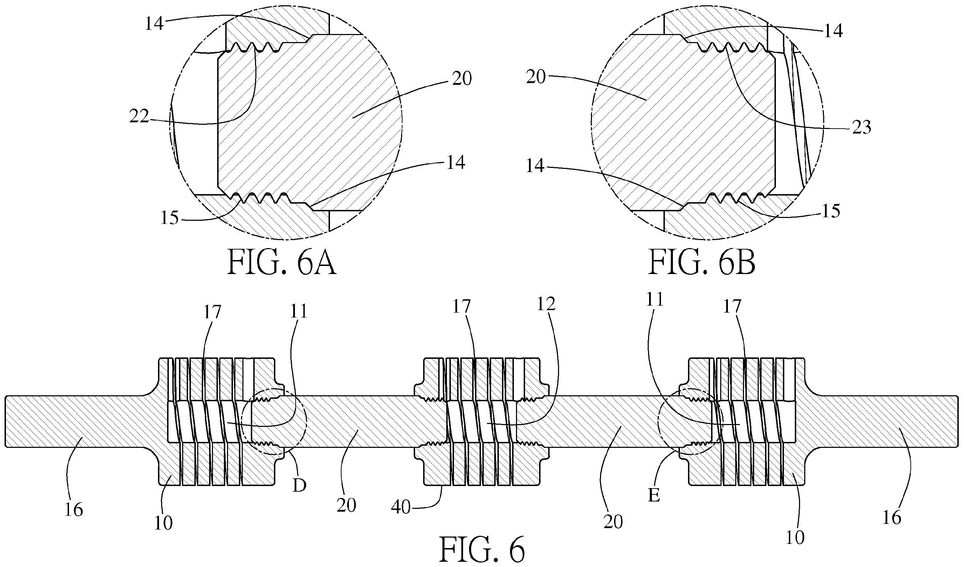

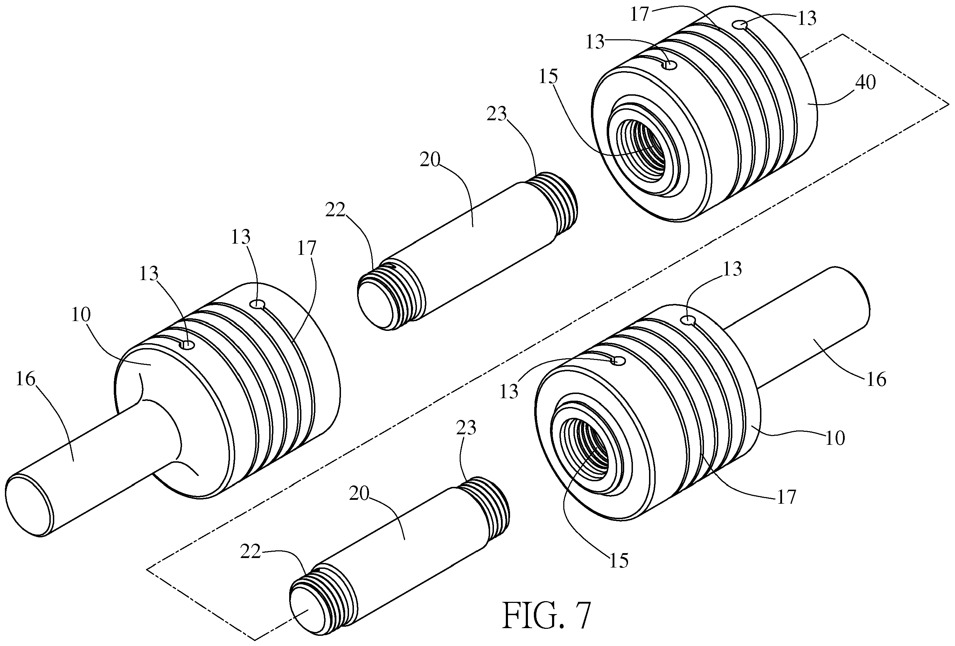

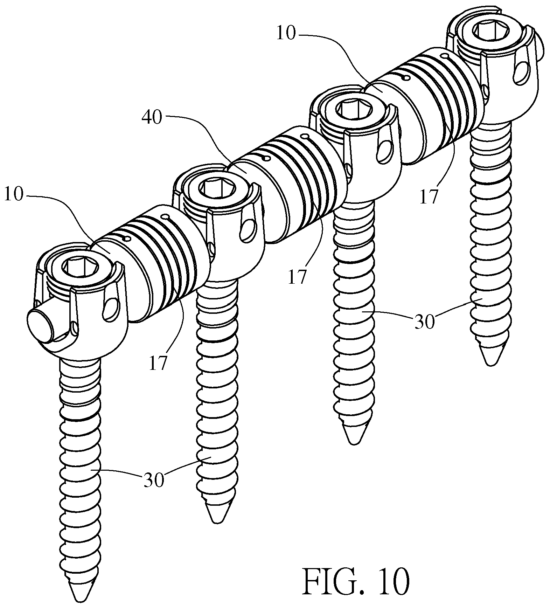

Referring to FIGS. 5, 6, 7, and 10, a spring elastic device in accordance with a second preferred embodiment of the invention is shown. The characteristics of the resilient connecting members 10 and the threaded connecting rod 20 in the second preferred embodiment are substantially the same as that previously described. In detail, the second preferred embodiment comprises two resilient connecting members 10, two threaded connecting rods 20, and a resilient connecting element 40. Each resilient connecting member 10 includes an externally threaded cylinder 17 having two radial holes 13 at two ends of the threads respectively, an axial hole 11 having a flat or inclined bottom, an internally threaded portion 15 formed at an open end of the axial hole 11, and a cylindrical connection rod 16 integrally formed with the externally threaded cylinder 17 and projecting out of an end of the externally threaded cylinder 17 opposing the open end of the axial hole 11.

The resilient connecting element 40 includes an externally threaded cylinder 17 having two radial holes 13 at two ends of the threads respectively, an axial channel 12, and two internally threaded portions 15 formed at two open ends of the channel 12 respectively.

The threaded connecting rod 20 includes a first externally threaded portion 22 at a first end, and a second externally threaded portion 23 at a second end opposing the first end. The first externally threaded portion 22 is secured to the internally threaded portion 15 of one resilient connecting member 10 or one internally threaded portion 15 of the resilient connecting element 40. The second externally threaded portion 23 is secured to the internally threaded portion 15 of the other resilient connecting member 10 or the other internally threaded portion 15 of the resilient connecting element 40. As a result, the threaded connecting rods 20, the resilient connecting members 10, and the resilient connecting element 40 are fastened together. Four fastening members 30 are put on the threaded connecting rods 20, the connection rod 16 of one resilient connecting member 10, and the connection rod 16 of the other resilient connecting member 10 respectively.

Each of the threaded connecting rod 20 and the connection rod 16 has a diameter of 5.5mm. The threaded connecting rod 20 is solid as shown or hollow in another embodiment. The externally threaded cylinder 17 has a uniform diameter and has a diameter greater than that of the threaded connecting rod 20 or that of the connection rod 16. The mouth 14 of the internally threaded portion 15 of the resilient connecting member 10 (or the resilient connecting element 40) has a diameter gradually increased toward an open end of the externally threaded portion 15.

For extending, a plurality of resilient connecting elements 40 and more than two threaded connecting rods 20 are required in which each resilient connecting element 40 is secured to and between two adjacent threaded connecting rods 20, the leftmost threaded connecting rod 20 is secured to and between one resilient connecting member 10 and the leftmost resilient connecting element 40, and the rightmost threaded connecting rod 20 is secured to and between the other resilient connecting member 10 and the rightmost resilient connecting element 40 by threading in a manner discussed above.

The invention has the following characteristics: No core tube is required, thereby eliminating the problems of core tube breaks or permanent distortion which could make it impossible for the externally threaded cylinder 17 to return to its original shape. No sealing members are required to seal two ends of the resilient connecting member 10, thereby eliminating the problem of looseness. The externally threaded cylinder 17 has a uniform diameter and has a diameter greater than that of the threaded connecting rod 20 so that the structural strength of the externally threaded cylinder 17 can be increased. Further, the provision of the two radial holes 13 at two ends of the threads of the externally threaded cylinder 17 respectively can prevent the externally threaded cylinder 17 from breaking. The axial hole 11 or the channel 12 of the externally threaded cylinder 17 has a smaller diameter so that it is not limited by the length of the threaded connecting rod 20. Further, less restriction is imposed on extension or retraction of the whole. Finally, the threaded connecting rod 20 can be solid or hollow when rigidity is considered.

## Claims

1. A spinal spring elastic rod device comprising:
at least one resilient connecting member (10); and
at least one threaded connecting rod (20);
wherein each of the at least one resilient connecting member (10) includes an externally threaded cylinder (17) having an axial hole (11) with an internally threaded portion (15) formed at an open end of the axial hole (11) and further comprises a connection rod (16) projecting out of an end of the externally threaded cylinder (17) opposing the open end of the axial hole (11), wherein the connection rod (16) is integrally formed with the externally threaded cylinder (17);
wherein each of the at least one threaded connecting rod (20) includes a first externally threaded portion (22) at a first end; and
wherein the externally threaded cylinder (17) has a diameter greater than that of each of the at least one threaded connecting rod (20),
**characterized in that**
two resilient connecting members (10) are provided, and
each of the at least one threaded connecting rod (20) includes a second externally threaded portion (23) at a second end opposing the first end, wherein the internally threaded portion (15) of each of the at least one first resilient connecting member (10) is engageable with one of the externally threaded portions (22, 23) respectively.

2. The spinal spring elastic rod device of claim 1, wherein the axial hole (11) has a bottom which is flat or inclined.

3. The spinal spring elastic rod device of claim 1, wherein at least one resilient connecting element (40) is additionally provided, in which the axial hole (11) is through to form an axial channel (12) including two internally threaded portions (15) formed at two open ends thereof respectively.

4. The spinal spring elastic rod device of claim 1, wherein each of the threaded connecting rod (20) is solid or hollow.

5. The spinal spring elastic rod device of claim 1, wherein the externally threaded cylinder (17) has two radial holes (13) at two ends of the threads thereof respectively.

6. The spinal spring elastic rod device of claim 1, wherein a mouth (14) of the internally threaded portion (15) of the axial hole (11) has a diameter gradually increased toward an open end thereof.

7. The spinal spring elastic rod device of claim 1, further comprising at least one fastening member (30) each put on the corresponding threaded connecting rod (20).

## Patentansprüche

1. Spinale federelastische Stangenvorrichtung, die Folgendes umfasst:
mindestens ein nachgiebiges verbindendes Element (10) und
mindestens eine verbindende Gewindestange (20),
wobei jedes des mindestens einen nachgiebigen verbindenden Elements (10) einen Zylinder (17) mit Außengewinde beinhaltet, der ein axiales Loch (11) mit einem Abschnitt (15) mit Innengewinde aufweist, der an einem offenen Ende des axialen Lochs (11) ausgebildet ist, und ferner eine Verbindungsstange (16) umfasst, die über ein Ende des Zylinders (17) mit Außengewinde vorspringt, das dem offenen Ende des axialen Lochs (11) gegenüberliegt, wobei die Verbindungsstange (16) einteilig mit dem Zylinder (17) mit Außengewinde ausgebildet ist,
wobei jede der mindestens einen verbindenden Gewindestangen (20) einen ersten Abschnitt (22) mit Außengewinde an einem ersten Ende beinhaltet und
wobei der Zylinder (17) mit Außengewinde einen Durchmesser aufweist, der größer als der Durchmesser jeder der mindestens einen verbindenden Gewindestange (20) ist,
**dadurch gekennzeichnet, dass**
zwei nachgiebige verbindende Elemente (10) vorgesehen sind und
jede der mindestens einen verbindenden Gewindestangen (20) einen zweiten Abschnitt (23) mit Außengewinde an einem zweiten Ende beinhaltet, das dem ersten Ende gegenüberliegt, wobei der Abschnitt (15) mit Innengewinde jedes des mindestens einen ersten nachgiebigen verbindenden Elements (10) jeweils mit einem der Abschnitte (22, 23) mit Außengewinde in Eingriff bringbar ist.

2. Spinale federelastische Stangenvorrichtung nach Anspruch 1, bei der das axiale Loch (11) einen Boden aufweist, der flach oder geneigt ist.

3. Spinale federelastische Stangenvorrichtung nach Anspruch 1, bei der zusätzlich mindestens ein nachgiebiges verbindendes Element (40) vorgesehen ist, durch das das axiale Loch (11) geht, um einen axialen Kanal (12) auszubilden, der zwei Abschnitte (15) mit Innengewinde beinhaltet, die jeweils an zwei offenen Enden davon ausgebildet sind.

4. Spinale federelastische Stangenvorrichtung nach Anspruch 1, bei der jede der verbindenden Gewindestangen (20) massiv oder hohl ist.

5. Spinale federelastische Stangenvorrichtung nach Anspruch 1, bei der der Zylinder (17) mit Außengewinde entsprechend zwei radiale Löcher (13) an zwei Enden des Gewindes davon aufweist.

6. Spinale federelastische Stangenvorrichtung nach Anspruch 1, bei der eine Öffnung (14) des Abschnitts (15) mit innengewinde des axialen Lochs (11) einen Durchmesser aufweist, der schrittweise zu einem offenen Ende davon hin zunimmt.

7. Spinale federelastische Stangenvorrichtung nach Anspruch 1, die ferner mindestens ein Befestigungselement (30) umfasst, wobei jedes auf die entsprechende verbindende Gewindestange (20) aufgesetzt ist.

## Revendications

1. Dispositif de bielle élastique à ressort rachidien comprenant:
au moins un organe de raccordement résilient (10); et
au moins une bielle filetée (20);
dans lequel chacun de l'au moins un organe de raccordement résilient (10) inclut un cylindre à filets externes (17) ayant un trou axial (11) avec une portion à filets internes (15) formée au niveau d'une extrémité ouverte du trou axial (11) et comprend en outre une bielle (16) dépassant d'une extrémité du cylindre à filets externes (17) opposée à l'extrémité ouverte du trou axial (11),
dans lequel la bielle (16) est formée solidairement avec le cylindre à filets externes (17);
dans lequel chacune de l'au moins une bielle filetée (20) inclut une première portion à filets externes (22) à une première extrémité; et
dans lequel le cylindre à filets externes (17) a un diamètre plus grand que celui de chacun de l'au moins une bielle filetée (20),
**caractérisé en ce que**
deux organes de raccordement résilients (10) sont prévus, et
chacune de l'au moins une bielle filetée (20) inclut une seconde portion à filets externes (23) à une seconde extrémité opposée à la première extrémité, dans lequel la portion à filets internes (15) de chacun de l'au moins un premier organe de raccordement résilient (10) peut être mis en prise avec l'une des portions à filets externes (22, 23) respectivement

2. Dispositif de bielle élastique à ressort rachidien selon la revendication 1, dans lequel le trou axial (11) a un fond qui est plat ou incliné.

3. Dispositif de bielle élastique à ressort rachidien selon la revendication 1, dans lequel au moins un élément de raccordement résilient (40) est de surcroît prévu, dans lequel le trou axial (11) est traversant pour former un canal axial (12) incluant deux portions à filets internes (15) formées à deux extrémités ouvertes de ces dernières respectivement.

4. Dispositif de bielle élastique à ressort rachidien selon la revendication 1, dans lequel chacune des bielles filetées (20) est pleine ou creuse.

5. Dispositif de bielle élastique à ressort rachidien selon la revendication 1, dans lequel le cylindre à filets externes (17) comporte deux trous radiaux (13) à deux extrémités des filets de ces derniers respectivement.

6. Dispositif de bielle élastique à ressort rachidien selon la revendication 1, dans lequel une bouche (14) de la portion à filets internes (15) du trou axial (11) a un diamètre augmentant progressivement vers une extrémité ouverte de ce dernier.

7. Dispositif de bielle élastique à ressort rachidien selon la revendication 1, comprenant en outre au moins un organe d'arrimage (30) chacun placé sur la bielle filetée (20) correspondante.
